Europäisches Patentamt

European Patent Office

Office européen des brevets

(19)

(11) Publication number: **0 413 120 A2**

# EUROPEAN PATENT APPLICATION

(12)

(21) Application number: **90112686.2**

(22) Date of filing: **03.07.90**

(51) Int. Cl.⁵: **A61K 31/60, A61K 9/50,**
**A61K 9/16**

(30) Priority: **16.08.89 US 394683**

(43) Date of publication of application:
**20.02.91 Bulletin 91/08**

(84) Designated Contracting States:
**AT BE CH DE DK ES FR GB GR IT LI NL SE**

(71) Applicant: **STERLING DRUG INC.**
**90 Park Avenue**
**New York New York(US)**

(72) Inventor: **Dressman, Jennifer Boehm**
**501 Detroit Street, No. 1**
**Ann Arbor, Michigan(US)**
Inventor: **Monkhouse, Donald Charles**
**439 King of Prussia Road**
**Radnor, Pennsylvania(US)**

(74) Representative: **Baillie, Iain Cameron et al**
**c/o Ladas & Parry Isartorplatz 5**
**D-8000 München 2(DE)**

(54) Aspirin granules with gastroprotectant coating.

(57) Discrete aspirin granules coated with a gastroprotectant coating such that the coated granules have a particle size less than 250μm.

EP 0 413 120 A2

The invention relates to a rapid-onset, gastroprotectant, coated aspirin formulation.

Aspirin (acetylsalicylic acid) is widely used for its analgesic, antipyretic, antiinflammatory, and antithrombotic properties. One major drawback of the drug is, however, its propensity to induce gastric irritation. In order to reduce gastric irritation, enteric-coated formulations of aspirin have been devised which prevent dissolution of the drug in the acid environment of the stomach. However, currently marketed enteric-coated aspirin preparations are characterized by prolonged delay between drug ingestion and achievement of therapeutic blood concentrations. Such delays render these formulations less suited for use in conditions where rapid analgesia is required, e.g. to alleviate headache.

Evidence suggests that the delayed appearance of drug in the blood stream following ingestion of enteric-coated aspirin is related to the time required for this form to pass through the stomach to more distally located absorptive sites where the enteric coating dissolves. This contrasts with the more rapid appearance in the blood of the conventional, uncoated form.

Minami and McCallum [ Gastroenterology 86 1592 - 1610 (1984)] showed that emptying of aqueous solutions from the human stomach followed a logarithmic decay curve. Five hundred mL of isotonic saline solution, a neutral, isoosmolar, calorically inert liquid, was 50% eliminated at 12 minutes. In contrast, digestable solids are emptied from the stomach only when they have been changed to an essentially liquified form; solid particles are retained in the stomach until they are less than 2 mm in size. Indigestible solids larger than 2-3 mm in diameter are not completely emptied by the stomach in the fed state; in the fasted state they are cleared approximately every two hours by the interdigestive myoelectric complex.

It is known that, for drugs taken in granular form, transit time through the stomach of dogs is related to particle size. Meyer et al. [ Gastroenterology 89 , 805-813 (1985)] showed in dogs that particles with neutral bouyancy taken with food emptied progressively faster as their diameters decreased from 5 mm to 1 mm, but 0.015 mm spheres emptied at about the same rate as those with diameters of 1 mm.

Meyer et al. [ Gastroenterology 94 , 1315-1325 (1988)] stated that in humans 1 mm spheres emptied consistently faster than 2.4 or 3.2 mm spheres when ingested together with food. They concluded that in the 1 - 3 mm range of diameters, sphere size was a more important determinant of sphere emptying than was meal size. They did not examine particles smaller than 1 mm diameter. They state:

> "Our previous canine study indicated a size-response curve in which 0.015 mm and 1 mm spheres emptied at about the same rate, but spheres 1 to 5 mm in diameter emptied at slower rates as the sphere diameters increased. By contrast, a continuous size response curve between 1 and 3.2 mm was not so evident in these human studies; although in individual studies 1 mm spheres emptied faster than 2.4 or 3.2 mm spheres, areas under the curves or 50% emptying times were about the same for 1.6, 2.4, and 3.2 mm: spheres. From these data one gets the impression that there is a discreet, single threshold between 1 and 1.6 mm that separates faster passage of 1 and 0.5 mm spheres from the similarly slower passage of 1.6 to 3.2 mm spheres."

In contrast to Meyer's results in dogs, we have surprisingly discovered that there does not appear to be a threshold around 1 mm in humans, but rather that enteric-coated aspirin granules of particle sizes below 1 mm show a decreasing time to peak serum salicylate concentration as a function of decreasing granule size. And, indeed, granules of particle size less than 250 $\mu$m (0.25 mm) approach uncoated aspirin in rapidity of attainment of peak serum salicylate concentration. We believe this rapid attainment of peak serum concentration is a reflection of the rapid gastric emptying (approaching that of liquids) of the very small particles.

The enteric-coating of aspirin granules is known in the art, and, in fact, at least two products based on enteric-coated granules of aspirin were formerly commercially available in the United States. However, none of the prior art recognizes the importance of particle sizes below 250$\mu$m for rapid onset. Nor are any enteric-coated aspirin granules of particle size below 250$\mu$m described in the prior art. The two discontinued enteric-coated granular aspirin products, Ecotrin® and Encaprin® both consisted of granules having a particle size range from 500 to 1200$\mu$m.

Appelgren et al. [U.S. Patent 4,562,061] describe the coating of aspirin granules of particle size 500 to 1200 $\mu$m with methyl esterified methacrylic acid polymers (Eudragit®L and Endragit®S) and a plasticizer selected from fatty alcohols (cetanol) and fatty acids (stearic acid). The object of the invention is to release aspirin distally in the small intestine (column 1 line 53 - 55). Gastric residence time is not considered or examined.

Guy et al. [U.S. Patent 3,906,086] describe the coating of aspirin granules with an enteric-coating of cellulose acetate phthalate for the purpose of timed release. The aspirin granules are ground to 20 mesh (840 $\mu$m).

Ying [PCT Application 88/01506] describes the coating of vitamin $B_2$ microgranules with butyl

methacrylate containing dibutylphthalate and stearic acid. The average size of the granules is described as "less than 500μm" (page 13 line 20 to line 21); the lower limit of particle size is not disclosed but the generic disclosure indicates that "microgranules of a very small particle size, in the order of 50μm to 500μm, may be produced according to the practice of the present invention" (page 5 line 19 to line 21). Ying suggests (page 2 line 10 to line 13) that the method can be extended to aspirin, but the process of fabrication of the granules requires an aqueous solution of the medicament and would be expected to produce unacceptable hydrolysis of aspirin.

Valenti [U.S. Patent 4,766,012] describes a coacervation process for microencapsulation with enteric polymers to produce enteric-coated granules of mean particle size less than 75μm. The process requires dissolving the enteric coating agent in water by adding base, dispersing the medicament in water and combining the two. Aspirin would be expected to undergo significant hydrolysis during this process and, in fact, aspirin is not among the antiinflammatory and analgesic medicaments described as suitable substrates for the invention (column 4, line 18 to line 21).

Kitajima et al. [U.S. Patent 3,703,576] describe a coacervation process for making aspirin granules of 300 to 1500 μm particle size coated with ethylcellulose. Ethylcellulose is not an enteric coating and the object of the invention appears to have been to provide extended duration rather than rapid onset (column 3 line 19 to line 24). The coacervation technique used with ethylcellulose would not be expected to be applicable in the case of enteric-coating polymers, which have very different solubility from that of ethylcellulose. Kitajima et al. [U.S. Patent 3,951,851] describe a second coacervation process for coating aspirin with ethylcellulose to provide particles of about 300 to about 500 μm diameter.

Holliday et al. [U.S. Patent 3,524,910] describe a sustained relief analgesic composition comprising granules of aspirin coated with ethylcellulose. The aspirin before coating has a particle size such that "substantially all particles are caught on a screen having 0.149 mm openings, a major portion are caught on a screen having 0.250 mm openings, and substantially all will pass a screen of 0.841 mm openings." (Column 6, line 57 to 61.) "The thinness of the ethyl cellulose coating is such that the size of the ethyl cellulose encapsulated aspirin particles is essentially the same as the size of the uncoated aspirin particles." (Column 2, line 59 to 62.) As above, the coating is not an enteric coating.

Hsiao [U.S. Patent 4,555,399] describes the coating of aspirin crystals with ethylcellulose and hydroxypropylcellulose to yield coated granules of "about 40 mesh" (420 μm). The combination of ethylcellulose and hydroxypropylcellulose is not an enteric-coating and in fact Hsiao teaches away from enteric-coating: "Enteric-coated formulations are not suitable for persons requiring the fast onset of aspirin's analgesic/-antipyretic activity, eg, rapid relief from headache, pain/fever." (Column 1 line 18 to line 21.) He continues, "The present invention provides an aspirin tablet, which, when ingested, rapidly disintegrates in the stomach into numerous polymer-coated aspirin crystals whereby the aspirin is absorbed in the stomach [underlining added] with minimal contact with the gastric mucosa." (column 1 line 47 to line 51.) He suggests that the invention may be extended to aspirin crystals having a particle size distribution ranging from about 20 to about 60 mesh (250 μm to 841 μm), with 40 mesh (420 μm) preferred. Hsiao [U.S. Patent 4,508,702] describes a very similar invention comprising aspirin of particle size to 30 to 60 mesh (250 μm. to 595 μm) coated with ethyl cellulose and hydroxypropylcellulose to yield a particle whose final size is undisclosed.

Boncey et al. [U.S. Patent 3,882,228 and its divisional, 3,887,700] describe aspirin granules coated with water-soluble coatings such as mannitol, lactose, and sorbitol by a spray drying process. The resulting granules have a mean particle size less than 75 μm, but once again the process is not suited to enteric coatings.

Alpsten et al. [ European Journal of Clinical Pharmacology 22 , 57-61 (1982)] describe a study of gastric emptying of enteric-coated and uncoated microgranules of aspirin of particle size 500 to 1000 μm. Gastric emptying time of uncoated and enteric-coated granules was not significantly different, with 50% being eliminated in about 1 hour. However, the absorption of acetylsalicylic acid from the uncoated granules occurred in parallel with the gastric emptying whereas the absorption from the enteric-coated granules was delayed for about 3 hours after gastric emptying. The authors concluded that the slow absorption of acetylsalicylic acid from enteric-coated granules could be explained partly by granule gastric emptying and partly by slow dissolution of the aspirin granules in the intestine. They state that "gastric emptying was almost complete within 3.5 hours and cannot therefore entirely explain the delayed absorption, [of enteric-coated granules] which lasted for at least 6 hours. The delayed absorption indicates that the dissolution rate of the enteric-coated ASA granules was slower than that observed in vitro, when 90% of the granules dissolved in simulated intestinal fluid (pH 6.5) within 30 minutes."

Bogentoft et al. [ European Journal of Clinical Pharmacology 14 , 351-355 (1978)] describe the plasma salicylic acid levels in humans following ingestion of enteric-coated granules (Reumyl®) and conventional tablets (Bayer®) as a function of time. The peak plasma salicylate levels were reached at 6 hours for the enteric-coated granules versus 2 hours for the conventional uncoated aspirin.

Thus none of the above cited references teach the desirability of making enteric-coated aspirin granules of less than 500 μm, none of the references that describe aspirin of particle size less than 500 μm describe enteric coatings, and none of the reference teachings of enteric coatings on small particles can be applied to aspirin.

## SUMMARY OF THE INVENTION

The invention relates to discrete aspirin granules coated with a gastroprotectant coating such that the coated granules have a particle size less than about 250 μm. By gastroprotectant coating it is meant that the coating material is substantially insoluble in aqueous solutions below pH 5.0, but it becomes substantially soluble in aqueous solutions at some pH between about 5.0 and about 7.5. Since the median fasting duodenal pH in humans is 6.1, a coating that begins to dissolve at about pH 5.0 and that is substantially soluble at about pH 6.0 is preferred because it will dissolve readily upon entering the duodenum of most patients. By gastroprotectant coating it is further meant that the coating is of such thickness that the granules release less than 30% of their aspirin in 2 hours at pH 1.2 under standard USP test conditions for enteric coatings, while still releasing aspirin in the intestine rapidly enough to provide peak serum salicylic acid levels within three hours in fasted patients.

The requirement that intact granules release very little of their aspirin in the stomach leads to a need for a certain minimum thickness for the enteric coat. The thickness depends upon the chemical and mechanical attributes of the particular material chosen for the coating. With present enteric polymer technology this minimum coating thickness, combined with the geometric ratio between sphere diameter and sphere volume, results in an optimum particle size between about 75 and 150 μm. According to our studies, the smaller the particle size, the better; however, below about 75 μm the ratio of the weight of enteric coating to the weight of medicament becomes so large as to render the manufacture of the particle economically unattractive. Further, the volume of the coating material, as a percentage of the volume of any dosage form made from the coated granules, becomes limiting because at some point the average human patient can no longer swallow the dosage form containing a therapeutically effective amount of aspirin.

A further aspect of the invention relates to a rapid onset dosage form comprising the above-described coated aspirin granules of less than about 250 μm diameter. Such dosage forms may be, for example, tablets or capsules, and either is within the scope of the invention. For analgesia or antipyresis in humans the preferred dosage form contains between 50 and 650 mg of aspirin, preferably about 325 mg.

One can provide a rapid-onset analgesic or antipyretic effect in humans by orally administering a dosage form containing the above-described coated granules.

The invention resides in aspirin granules coated with a gastroprotectant coating such that the coated granules have a particle size less than about 250 μm. The preferred coatings may be chosen from those well-known in the art as enteric coating agents. Particularly preferred are cellulose acetate phthalate, poly-(vinylacetate phthalate), hydroxypropylmethylcellulose phthalate, or a 1:1 copolymer of ethyl acrylate and methacrylic acid having an average molecular weight of about 250,000. The acrylate polymer is commercially available as an aqueous dispersion from Rohm Pharma (West Germany) under the tradename of Eudragit® L30D; cellulose acetate phthalate is commercially available from Eastman, FMC and Polysciences (USA). An aqueous dispersion of poly(vinyl acetate phthalate) is available from Colorcon (USA) as Coateric®. Hydroxypropylmethylcellulose phthalate is available from Shin-Etsu (Japan) as HP-50 and HP-55. Plasticizers, for example triacetin or polyethylene glycol, may optionally be incorporated in the gastroprotectant coat as may antiadherents such as talc, silica or titanium dioxide. The weight of coating may be from about 10% to about 75%, preferably about 25% to about 40% more preferably about 30 to about 35% of the weight of the coated granule.

A preferred embodiment of the granule consists of cuboidal aspirin crystals of about 55 to about 220 μm, most preferably 55 to 130 μm average particle size, which are then coated with acrylate/methacrylic acid copolymer, containing from 5 to 25, preferably 10, weight percent triacetin. The thickness of the coating must be sufficient to minimize dissolution of the coated granule in the stomach during the residence time of the granule in the stomach. With the coatings of the preferred embodiment, the weight of the coating comprises from about 10% to about 75% preferably from about 30% to about 35%, of the weight of the coated granule. The lower limit is presently constrained by the minimum effective coating thickness of available enteric coatings; the upper limit is controlled by a patient's ability to ingest the volume of granules

4

necessary to provide and efficacious quantity of aspirin.

Many commercially available forms of aspirin are crystalline rods or needles having a ratio of length to thickness as high as 5:1. Such elongated particles are difficult to coat evenly and have a tendency to fracture after coating, thus exposing uncoated aspirin. Those elongated rods that are successfully coated and do not subsequently fracture may still lead to anomalous effects in gastric emptying. For these reasons we prefer so-called "cuboidal" crystals as the starting material. Cuboidal crystals, of which one commercial source is Chemische Fabrik Aubing (West Germany), have a preponderance of particles whose greatest dimension is not more than twice their second dimension and not more than four times their smallest dimension.

Alternatively, aspirin in the standard form of rods or needles may be milled and air classified to provide particles of the proper dimensions, but there is a high proportion of fines and the process is more wasteful of starting material.

A method of preparing aspirin in accordance with our invention comprises the spray-coating of aspirin granules, preferably cuboidal granules, preferably of particle size about 55 to about 220 $\mu$m, with a suspension of about 40 to about 60, preferably about 50, parts of 30% aqueous dispersion of a copolymer of an acrylic acid and an acrylate, preferably a 1:1 copolymer of methacrylic acid and ethyl acrylate of average molecular weight of about 250,000, and from about 1 to about 20, preferably about 1.5, parts of a plasticizer, such as polyethylene glycol, glycerin, propylene glycol, a fatty alcohol, fatty acid, an acetyl trialkyl citrate, or, preferably, triacetin in about 48 parts of water. Although not necessary or preferred, from zero to about 45 parts of an antiadherent such as talc, silica, or titanium dioxide may be added.

The coating suspension may be applied using a fluidized bed coater at 23° to 65°C. with the volume and humidity of process air and the rate of spray adjusted such that agglomeration of the particles is avoided. The coating weight after drying may be 10% to 75%, preferably about 32%, of the weight of the coated particle. The particles are dried at 35° to 65°C., preferably about 60°C., until the coated particles contain less than 2% solvent, and passed though a sieve of appropriate dimension.

Alternatively it is contemplated that the spray-coating of aspirin granules can be carried out in similar fashion utilizing a solution of cellulose acetate phthalate, polyvinyl acetate phthalate, or hydroxypropylmethylcellulose phthalate in an organic solvent, although this process would be less preferred because of the environmental and safety concerns associated with the use of organic solvents.

## EXAMPLE 1

Aspirin (Aubing type 100/300) was sieved to provide 3.00 kg of granules that pass through a 60 mesh sieve and are retained on an 80 mesh sieve. Although the 80 mesh sieve has 180 $\mu$m openings, the static charge of uncoated aspirin results in much finer particles being retained.

A coating solution was prepared by mixing 5.00 kg of Eudragit®. L30D in 4.84 kg of water and adding 0.15 kg of triacetin. The mixture was stirred with low agitation for 15 minutes. A Glatt GPCG-5 fluidized bed coater, equipped with a 7-inch Wurster column was preheated to 60°C. The column was charged with 3.00 kg of the aspirin crystals of 70 to 250$\mu$ particle size and fluidized to an appropriate level (about 80 cu. ft./min). The coating suspension was applied through a 1.2 mm nozzle, atomized by 4.0 bar air pressure, at a rate of 20 g per minute to maintain an outlet air temperature of about 35°C. When the coating suspension was completely consumed (about 8.5 hours), the material was dried in the coater at 60°C. inlet air temperature until a "loss on drying" test showed less than 2% moisture. The granules were passed through a 100 mesh screen and retained on a 200 mesh screen. The resulting granules were comprised of 64.5 wt.% aspirin, 32.2 wt.% Eudragit® L and 3.22 wt.% triacetin. Their size range after coating and final sieving was 75 to 150 $\mu$m with a mean particle size of about 100 $\mu$m.

When the granules were tested according to the procedure of USP XXI page 1245 in 0.1M HCl, 25% of the aspirin was released in 2 hours; in stage II of the USP test, 100% was released within 15 minutes at pH 6.8 in phosphate buffer.

Bioavailability was examined by comparing the granules of the invention with conventional (Reumyl®) enteric-coated granules of size range 500 to 1200 $\mu$m in eight, healthy, male, fasted volunteers in a four-way crossover study. Volunteers were given a single dose of test granules containing 325 mg of aspirin dispersed in 30 mL of apple juice, followed by 200 mL of water. Blood samples were collected at 20 min., 40 min., 1 hour, 1.5 hours, 2 hours, 2.5 hours; and each hour thereafter to 24 hours. The samples after perchloric acid treatment were examined by HPLC with UV detection at 300 nm. The minimum quantifiable level of salicylic acid in this assay was 2.5 $\mu$g/mL. The individual plasma concentration versus time profiles were then examined to derive the peak concentration ($C_{max}$) and the time to reach peak concentration ($t_{max}$). The median time in hours to peak concentration ($t_{max}$) and the range for $t_{max}$ are shown in Table 1.

A second bioavailability study was performed comparing the granules of the invention with a conventional, uncoated aspirin in twelve healthy, male, fasted volunteers in a four-way randomized crossover study. The protocol was the same as in the preceding study except that the conventional aspirin was administered as a tablet. The $t_{max}$ and range are shown in Table 1.

The protocol of the first bioavailability study was also utilized to examine the effect of food on $t_{max}$. The eight volunteers were given a single dose of test granules containing 325 mg of aspirin in 30 mL of apple juice followed by 200 mL of water. The aspirin was taken with a standardized breakfast. The median time in hours to peak concentration, $t_{max}$, and the range for $t_{max}$ for the conventional granules and the granules of the invention are shown in Table 2.

Table 1

| Median Time (Hrs.) to Reach Peak Salicylic Acid Concentration in Fasted Volunteers | | |
|---|---|---|
| Treatment | Study 1 (n = 8) | Study 2 (n = 12) |
| Conventional granules (500-1200μm) | 6.5(5.0-8.0) | - |
| Example 1 granules (<250μm) | 2.5(2.0-3.0) | 2.5(1.5-3.0) |
| Bayer® aspirin (uncoated tablet) | - | 1.5 (0.67-2.0) |

As can be seen from Table 1, the coated granules of the invention provide much more rapid achievement of maximum serum levels of salicylic acid (medium $t_{max}$ = 2.5 hrs) than conventional (500-1200$\mu$) enteric-coated granules (median $t_{max}$ = 6.5 hrs); the time to peak levels seen for the granules of the invention approaches that seen for conventional, uncoated aspirin (median $t_{max}$ = 1.5 hrs).

Table 2

| Median Time (Hrs.) to Reach Peak Salicylic Acid Concentration in Fed Volunteers | |
|---|---|
| Treatment | Study 1 (n = 8) |
| Conventional granules | 8.0(7.0-10.0) |
| Example 1 granules | 4.0(2.0-5.0) |

As can be seen from Table 2, the granules of the invention provide much more rapid achievement of maximum serum levels of salicylic acid than conventional granules when taken with food: The median $t_{max}$ is four hours faster for the granules of the invention, as was the case in fasted subjects.

The granules may be fabricated into rapid-onset dosage forms, such as capsules, caplets, or tablets, by processes well known in the art. For the production of tablets, the coated aspirin particles can be blended with commonly used pharmaceutical excipients such as microcrystalline cellulose, lactose (or other sugars), compressible starches, binders, flow agents and lubricants. These excipients can be direct-compression materials, used as received from the supplier, or they can be granulated, using a wet or dry method, to obtain a specific particule size prior to adding to the coated aspirin. The blended mixtures can be tabletted in accordance with typical procedures on a standard tablet compression machine. By rapid-onset dosage form is intended that the dosage form will disperse substantially completely within about thirty minutes in gastric fluid. Dosage forms, tablets or capsules, that do not rapidly disperse in the stomach would of course vitiate the advantages of the granules of the invention.

6

## Claims

1. Discrete aspirin granules coated with a gastroprotectant coating such that the coated granules have a particle size less than 250μm.

2. Discrete aspirin granules according to claim 1, having a mean particle size from about 75μm to about 150μm.

3. Discrete aspirin granules according to claim 1 or 2, wherein said gastroprotectant coating comprises from about 10% to about 75% of the weight of said granule.

4. Discrete aspirin granules according to claim 3, wherein the gastroprotectant coating comprises from about 30% to about 35% of the weight of said granule.

5. Discrete aspirin granules according to any one of claims 1-4, wherein the gastroprotectant coating is cellulose acetate phthalate, poly(vinyl acetate phthalate), hydroxypropylmethylcellulose phthalate or a copolymer of methacrylic acid and ethyl acrylate.

6. Discrete aspirin granules according to claim 5, wherein said gastroprotectant coating comprises a 1:1 copolymer of methacrylic acid and ethyl acrylate having an average molecular weight of about 250,000.

7. A rapid-onset dosage form comprising granules according to any one of the preceding claims.

8. A rapid-onset dosage form according to claim 7, containing from 50 to 650 mg of aspirin.

9. A rapid-onset dosage form according to claim 8, containing about 325 mg of aspirin.

10. The use of a compound according to any one of the preceding claims for preparing a medicament useful for treating humans in need of a rapid-onset analgesic or antipyretic effect.

Claims for the following Contracting States: Es, Gr

1. A process for preparing discrete aspirin granules characterized by coating aspirin granules with a gastroprotectant coating such that the coated granules have a particle size less than 250μm.

2. A process according to claim 1, wherein the resulting granules have a mean particle size from about 75μm to about 150μm.

3. A process according to claim 1 or 2, wherein said gastroprotectant coating comprises from about 10% to about 75% of the weight of said granule.

4. A process according to claim 3, wherein the resulting gastroprotectant coating comprises from about 30% to about 35% of the weight of said granule.

5. A process according to any one of claims 1-4, wherein the gastroprotectant coating is cellulose acetate phthalate, poly(vinyl acetate phthalate), hydroxypropylmethylcellulose phthalate or a copolymer of methacrylic acid and ethyl acrylate.

6. A process according to claim 5, wherein said gastroprotectant coating comprises a 1:1 copolymer of methacrylic acid and ethyl acrylate having an average molecular weight of about 250,000.

7. A process according to any one of the preceding claims, in which the granules are formulated into rapid-onset dosage form containing from 50 to 650 mg of aspirin.

8. A process according to claim 7, in which the resulting product contains about 325 mg of aspirin.

9. A process according to any one of the preceding claims, in which the coating is carried out by spraying.